# EUROPÄISCHE PATENTSCHRIFT

(11) **EP 4 058 137 B1**
(45) Veröffentlichungstag und Bekanntmachung des Hinweises auf die Patenterteilung: **22.10.2025**
(21) Anmeldenummer: 20820788.6
(22) Anmeldetag: 13.11.2020
(51) Int. Cl.: A61N 5/06, H04R 25/00

(54) **VORRICHTUNG ZUR VERBESSERTEN INDUZIERUNG VON SCHALL MITTELS ELEKTROMAGNETISCHER STRAHLUNG**
DEVICE FOR IMPROVED INDUCTION OF SOUND BY MEANS OF ELECTROMAGNETIC RADIATION
DISPOSITIF POUR L'INDUCTION AMÉLIORÉE DE SON AU MOYEN D'UN RAYONNEMENT ÉLECTROMAGNÉTIQUE

(30) Priorität: 15.11.2019 DE 102019130958
(43) Veröffentlichungstag der Anmeldung: 21.09.2022
(73) Patentinhaber: Universität des Saarlandes, 66123 Saarbrücken (DE)
(72) Erfinder: WENZEL, Gentiana Ioana Constanta, 66424 Homburg (DE); SCHICK, Bernhard, 36037 Fulda (DE); LANGENBUCHER, Achim, 66123 Saarbrücken (DE); KRUTTWIG, Klaus, 58099 Hagen (DE); ARZT, Eduard, 66123 Saarbrücken (DE)
(74) Vertreter: Schiweck Weinzierl Koch Patentanwälte Partnerschaft mbB
(86) Internationale Anmeldenummer: PCT/EP2020/081986
(87) Internationale Veröffentlichungsnummer: WO 2021/094499

(56) Entgegenhaltungen:
- EP-A1- 3 421 019
- WO-A1-2010/086452
- WO-A1-2010/086453
- WO-A1-93/10730
- WO-A2-2006/042298
- US-A- 5 925 453

## Beschreibung

Die Versorgung von Patienten mit einer Hörbehinderung unterschiedlicher Grade ist trotz aller Weiterentwicklungen von konventionellen und implantierbaren Hörprothesen unbefriedigend. Vor allem im Störgeräusch und für komplexe akustische Signale wie z.B. Musik können die heute verfügbaren Hörgeräte kein dem Natürlichen nahekommendes Hörvermögen bereitstellen und sind daher noch unbefriedigend. Diese Unzulänglichkeit heutiger Hörhilfen ist, unter anderem, durch die Schwierigkeit der erforderlichen sehr frequenzspezifischen Aktivierung des Hörsystems bedingt, welche mit den zurzeit zur Verfügung stehenden Hörhilfen noch nicht in ausreichendem Maße bewerkstelligt werden kann.

Eine grundsätzliche Alternative zu den mechanischen und elektrischen Stimulationsstrategien stellt die Aktivierung des Hörsystems mittels Licht dar. Die Besonderheit von Licht als Informationsübertragungsmedium ist, dass es sich dabei um eine Energieform handelt, welche sehr gezielt und streuungsarm appliziert werden kann. Dabei kann das Licht kontinuierlich in der Intensität oder Wellenlänge moduliert oder in gepulster Form appliziert werden. Grundsätzlich kann so eine optimierte Aktivierung des Hörsystems vorgenommen werden.

Eine gezielte direkte mechanische Stimulation definierter Abschnitte des Innenohrs mittels Laser-Pulsen ist bereits im Stand der Technik bekannt (z.B. "Optoacoustic induced vibrations within the inner ear"; Zhang KY, Wenzel Gl, Balster S, Lim HH, Lubatschowski H, Lenarz T, Ertmer W, Reuter G; Opt. Express, 2009 Dec 7;17(25):23037-43). Dazu wurde mit einem Nd:YAG-Laser im grünen Spektralbereich (532 nm) die Cochlea (das Innenohr) durch die Rundfenstermembran stimuliert. Dabei konnten das periphere Hörorgan aktiviert und entsprechende elektrische Signale sowohl vom Hirnstamm als auch von der zentralen Hörbahn abgeleitet werden. Die Amplitude der neuronalen Antworten konnte entsprechend der applizierten Pulsenergie moduliert werden. Weitere Untersuchungen ergaben, dass auch die Applikation von Lichtpulsen im Mittelohr und Gehörgang mit Parametern, die einen optoakustischen Effekt induzieren können, das Hörorgan aktivieren.

Ein weiteres Hörgerät, das ein Photoakustischeseffekt nutzt wird in WO 2010/086453 A1 beschrieben.

Ausgehend von diesen ersten grundsätzlich erfolgreich absolvierten Versuchen wurden verschiedene Parameter des Systems wie Wellenlänge, Laserpulsenergie, Pulswiederholungsfrequenz, Pulsdauer und Bestrahlungszielstruktur auf ihren optimalen Anwendungsbereich hin getestet mit dem übergeordneten Ziel der Herstellung optischer Hörprothesen. Für die Optimierung und Praktikabilität eines optischen Hörgerätes bedarf es einer optimierten Absorption der eingestrahlten Photonen in der Zielstruktur z.B. dem Trommelfell. Hierbei ist die grundlegende Rahmenbedingung, dass das gesunde Trommelfell zum größten Teil durchsichtig ist und daher nur einen Teil des applizierten Lichtes absorbiert.

Vor diesem Hintergrund kann die Aufgabe der Erfindung darin gesehen werden, die mechanische Anregung einer vibrierfähige Zielstruktur im menschlichen peripheren Hörorgan, insbesondere des Trommelfells, des Felsenbeins einschließlich des Mittelohres und des Innenohres, mittels Licht zu optimieren.

Die Aufgabe wird gelöst durch die Vorrichtung gemäß dem unabhängigen Patentanspruch sowie die Gegenstände der nebengeordneten Patentansprüche. Weitere vorteilhafte Ausgestaltungen finden sich in den abhängigen Patentansprüchen.

Der Erfindung basiert auf der Erkenntnis, dass eine Optimierung der Schallübertragung auf eine vibrierfähige Struktur des Hörorgans von Vorteil für die Herstellung optischer Hörgeräte wäre. Erfindungsgemäß kann durch Verwenden einer Vorrichtung, welche einen pigmentierten bzw. geeignet gefärbten Bereich und einen reflektierenden Bereich aufweist, die auf die Vorrichtung auftreffende Energie mit maximaler Leistung und minimalen Verlusten auf das biologische Gewebe, z.B. das Trommelfell übertragen werden. Zudem kann durch Applikation der erfindungsgemäßen Vorrichtung auf die biologische Struktur bei der Bestrahlung eine Schädigung des bestrahlten Gewebes und umliegender Strukturen vermieden werden. Die erfindungsgemäße Vorrichtung ist insgesamt geeignet, den in der biologischen Struktur optisch induzierten Schall zu unterstützen und ggfs. zu verstärken.

Erfindungsgemäß wird eine Vorrichtung bereitgestellt, insbesondere eine Vorrichtung zur verbesserten Induzierung von Schall mittels elektromagnetsicher Strahlung, welche eine Trägerschicht, eine erste Substanz bzw. ein erstes Material, welches bezüglich elektromagnetischer Strahlung mit einem vorbestimmen Wellenlängenspektrum eine reflektierende Eigenschaft aufweist, und eine zweite Substanz bzw. ein zweites Material aufweist, welches bezüglich elektromagnetischer Strahlung mit dem vorbestimmen Wellenlängenspektrum eine absorbierende Eigenschaft aufweist. Hierbei ist die erste Substanz in einem Bereich zwischen der Trägerschicht und der zweiten Substanz angeordnet. Die Trägerschicht kann mindestens eine Art von Elastomeren aufweisen.

Durch Anbringen der erfindungsgemäßen Struktur auf ein vibrierfähiges Gewebe, insbesondere auf das Trommelfell, das Felsenbein einschließlich Mittelohr und Innenohr, kann eine im Vergleich zur direkten Bestrahlung des vibrierfähigen Gewebes (also ohne der darauf angeordneten erfindungsgemäßen Vorrichtung) verbesserte optoakustische Stimulation des vibrierfähigen Gewebes erreicht werden. Bei der vorliegenden Vorrichtung handelt es sich um eine passive Struktur, welche ohne elektrische und elektronische Elemente auskommt. Während die zweite Substanz eine hohe Absorptionsfähigkeit hinsichtlich der Wellenlänge(n) der elektromagnetischen Strahlung aufweist, die für die optoakustische Anregung verwendet wird, beispielsweise Lasterstrahlung, hat die erste Substanz hinsichtlich der Wellenlänge(n) der elektromagnetischen Strahlung eine hohe Reflexionsfähigkeit. Bei der ersten Substanz kann es sich beispielsweise um Aluminium und/oder Silber oder eine Mischung daraus handeln, insbesondere um eine dünne Schicht daraus, welche auf der Trägerschicht angeordnet ist. Der reflektierende Bereich der Vorrichtung kann jedoch auch auf anderem Wege ausgebildet werden. Die erste Substanz kann auch eine Mehrzahl von dielektrischen Schichten umfassen, die einen wellenlängenselektiven Spiegel (dichroitische Spiegel) ausbilden hinsichtlich der Wellenlänge(n) der elektromagnetischen Strahlung. Bei der Verwendung von Laserlicht im optischen Bereich kann die erste Substanz über den gesamten sichtbaren Wellenlängenbereich oder Wesentliche Teile davon reflektierend sein.

Aus Sicht der Einfallsrichtung der elektromagnetischen Strahlung ist die zweite Substanz räumlich vor der ersten Substanz angeordnet, beispielsweise schichtweise. Die erste Substanz hat die Aufgabe, diejenige elektromagnetische Strahlung zu reflektieren, welche nicht von der zweiten Substanz absorbiert worden ist und verhindert damit eine Propagation der elektromagnetischen Strahlung durch die erfindungsgemäße Vorrichtung hindurch. Damit schützt die erfindungsgemäße Vorrichtung zugleich das vibrierfähige Gewebe vor der Einwirkung der elektromagnetischen Strahlung.

Bei der Trägerschicht kann es sich um ein flexibles Trägersubstrat handeln, dessen Oberfläche, die dem biologischen Gewebe zugewendet ist, adhäsiv ist. Alternativ kann ein Adhäsiv auf diese Oberfläche aufgetragen werden, um die Vorrichtung auf das biologische Gewebe anzubringen. Ferner kann die Trägerschicht aus einem biokompatiblen Material hergestellt sein, sodass die Vorrichtung problemlos auf das vibrierfähige Gewebe angebracht werden kann. Bei der Vorrichtung kann es sich um einen Patch handeln, welcher eine adhäsive/klebende Oberfläche aufweist zur Anbringung auf das vibrierfähige Gewebe. Die gesamte Vorrichtung kann eine Dicke im Bereich von etwa 80 µm bis etwa 2000 µm aufweisen, bevorzugt im Bereich von etwa 100 µm bis etwa 200 µm.

Bei der erfindungsgemäßen Vorrichtung kann es sich um eine durch Photonen aktivierbare Struktur für die Schallübertragung handeln, welche passiv, einfach strukturiert bzw. gezielt mikrostrukturiert ist und bei Anbringung auf das vibrierfähige Zielgewebe die Unterstützung der Schallübertragung auf das vibrierfähige Zielgewebe, etwa das Trommelfell, bei optischer Stimulation ermöglicht. Ferner kann durch die Anordnung der ersten Substanz auf der Trägerschicht (wobei eine Anordnung der ersten Substanz innerhalb der Trägerschicht auch möglich ist) und durch die Anordnung der zweiten Substanz auf bzw. über der ersten Substanz eine thermische Isolation zur Zielstruktur erreicht werden. Mit anderen Worten kann die zweite Substanz, welche zur optimalen Absorption der elektromagnetischen Strahlung eingerichtet ist, mindestens durch die transversale Dimension der Trägerschicht von dem vibrierfähige Zielgewebe isoliert sein, sodass ein Wärmeübertrag von der zweiten Substanz auf das vibrierfähige Zielgewebe minimiert werden kann.

Die grundlegende Funktionsweise der erfindungsgemäßen Vorrichtung beruht auf dem photoakustischen Effekt, durch den akustische Anregungen bzw. Wellen durch Absorption von elektromagnetischer Energie erzeugt werden. Die Absorption der elektromagnetischen Strahlung erfolgt dabei primär durch die zweite Substanz bzw. primär in einer die zweite Substanz enthaltenden Schicht. Der Energieeintrag führt zu einer thermischen Expansion innerhalb der der Vorrichtung, die in einer mechanischen Schwingung bzw. Anregung resultiert. Mit anderen Worten wird mittels der die elektromagnetische Strahlung absorbierenden zweiten Substanz die elektromagnetische (bevorzugt optische) Anregung in eine mechanische Anregung umgewandelt. Dieses ist insbesondere der Fall, wenn die Zeitskala der Transitzeit der durch Wärmeeinwirkung erzeugten Materialspannung durch die Vorrichtung größer ist als eine Pulsdauer der elektromagnetischen Strahlung, welche auf die Vorrichtung trifft. Das Vorliegen dieser Bedingung wird als "stress confinement" (Spannungseinsperrung) bezeichnet. Bei einer Platzierung der Vorrichtung auf dem Trommelfell als organische Zielstruktur kann diese bevorzugt in einem Bereich des Trommelfells über dem Umbo angeordnet werden, um eine gute mechanische Anregung des Trommelfells zu erreichen.

Gemäß weiteren Ausführungsformen der Vorrichtung kann die Trägerschicht zwei aneinander anliegende Schichten aufweisen. Generell kann die Dicke der ersten Schicht im Bereich von einigen Zehn Mikrometern liegen und beispielsweise 40 µm betragen. Generell kann die Dicke der zweiten Schicht ebenfalls im Bereich von einigen Zehn Mikrometern liegen, beispielsweise im Bereich von etwa 10 µm bis etwa 100 µm, bevorzugt im Bereich von etwa 40 µm bis etwa 80 µm.

Gemäß weiteren Ausführungsformen der Vorrichtung kann die erste Schicht der Trägerschicht ein Silikonelastomer, bevorzugt SSA MG 7-9800 aufweisen. Die zweite Schicht der Trägerschicht kann ebenfalls ein Silikonelastomer, bevorzugt Sylgard 184 aufweisen. Sylgard 184 besitzt einen hohen elastischen Anteil und zeigt ein relativ rigides Verhalten, wenn es auf einen Trägerfilm aufgebracht und polymerisiert wird. SSA MG 7-9800 zeichnet sich durch eine hohe viskose Komponente aus. Die erste SSA MG 7-9800 aufweisende Schicht kann zur Befestigung der Vorrichtung auf dem biologischen Gewebe, etwa dem Trommelfell, verwendet werden.

Gemäß weiteren Ausführungsformen der Vorrichtung kann die erste Substanz eine erste Funktionalschicht ausbilden oder in dieser enthalten sein, welche auf der Trägerschicht angeordnet ist. Damit kann die erste Substanz als reflektierende Schicht ausgebildet sein, welche auf der Trägerschicht ausgebildet ist. Die erste Substanz kann als Beschichtung auf der Trägerschicht oder auf einer Schicht, welche die zweite Substanz aufweist, angeordnet sein. Wie bereits erwähnt, kann die erste Substanz auch ein dielektrisches Material umfassen, welches in Form von dielektrischen dünnen Schichten vorliegt und so eine spiegelnde Schicht bereitstellt.

Gemäß weiteren Ausführungsformen der Vorrichtung kann die erste Funktionalschicht auf der zweiten Schicht der Trägerschicht angeordnet sein. Die zweite Schicht der Trägerschicht kann ihrerseits auf der ersten Schicht der Trägerschicht angeordnet sein, welche zur Anbringung an das vibrierfähige Gewebe dient.

Gemäß weiteren Ausführungsformen der Vorrichtung kann die zweite Substanz eine zweite Funktionalschicht bilden oder in dieser enthalten sein, welche auf der ersten Funktionalschicht angeordnet ist. Die erfindungsgemäße Vorrichtung kann eine Schichtstruktur aufweisen, wobei jede Schicht (Trägerschicht und die Funktionalschichten) ihre individuelle Dicke aufweisen kann. Die laterale Ausdehnung der ersten und der zweiten Funktionalschicht muss nicht der lateralen Ausdehnung der Trägerschicht entsprechen. Insbesondere kann die Trägerschicht größer sein als die darauf angeordneten Funktionalschichten sein. Die zweite Funktionalschicht kann insbesondere eine schwarz gefärbte oder pigmentierte Schicht aufweisen, welche elektromagnetische Strahlung absorbiert, beispielsweise im sichtbaren, nahultravioletten und nahinfraroten Bereich.

In weiteren Ausführungsformen wird ein Hörgerät bereitgestellt, welches die hier beschriebene Vorrichtung sowie einen Signalgeber aufweist, welcher eingerichtet ist, mittels mindestens einem Mikrophon Schall aufzunehmen und auf Basis des aufgenommenen Schalls elektromagnetische Strahlung mit dem vorbestimmen Wellenlängenspektrum zu emittieren. Die Emission der elektromagnetischen Strahlung kann dabei fokussiert in Richtung der Vorrichtung erfolgen. Die elektromagnetische Strahlung kann bevorzugt Laserlicht entsprechen und von einer Laserdiode emittiert werden. Folglich dient bei dem Hörgerät elektromagnetische Strahlung als Informationsübertragungsmedium zwischen dem Signalgeber und der Vorrichtung.

Erfindungsgemäß wird die hier beschriebene Vorrichtung zur Verwendung in einem Verfahren zur mechanischen Anregung des Trommelfells oder einer weiteren vibrierfähigen bzw. vibrierenden Struktur des Schädels, z.B. des Felsenbeins einschließlich Mittelohr und Innenohr, mittels elektromagnetischer Strahlung bereitgestellt.

Ferner wird eine Verwendung der hier beschriebenen Vorrichtung zur mechanischen Anregung des Trommelfells oder einer weiteren vibrierfähigen bzw. vibrierenden Struktur des Schädels bereitgestellt, wobei die Vorrichtung auf dem Trommelfell oder der weiteren vibrierfähigen Struktur des Schädels angebracht ist. Die mechanische Anregung erfolgt dabei durch Bestrahlung der Vorrichtung mit elektromagnetischer Strahlung, bevorzugt einem kollimierten oder fokussierten Strahl.

Ferner wird ein Verfahren zur mechanischen Anregung des Trommelfells oder einer weiteren vibrierfähigen Struktur des Schädels mittels elektromagnetischer Strahlung bereitgestellt, wobei das Verfahren Anbringen der hierin beschriebenen Vorrichtung auf das Trommelfell oder die weitere vibrierfähige Struktur des Schädels, und Bestrahlen der Vorrichtung mit elektromagnetischer Strahlung mit dem vorbestimmten Wellenlängenspektrum aufweist.

In weiteren Ausführungsformen des Verfahrens kann die elektromagnetische Strahlung in Form von modellierter bzw. modelierbarer Strahlung vorliegen. Beispielsweise kann die elektromagnetische Strahlung in Form eines amplitudenmodulierten Feldes vorliegen, welches eine Trägerfrequenz im Bereich von einigen Zehn Kilohertz oberhalb von etwa 20 kHz aufweist, beispielsweise 32 kHz, 50 kHz oder mehr.

Die beschriebenen Verwendung und Verfahren sind nicht Teil der Erfindung.

Die erfindungsgemäße Vorrichtung kann sowohl bei Tieren wie auch bei Menschen verwendet werden, um eine optoakustische Anregung des Trommelfells (oder der weiteren vibrierfähigen Struktur des Schädels) zu erreichen. Je nach Größe des Trommelfells kann die Dimension der Vorrichtung sowie beispielsweise ihr Gewischt daran angepasst werden. Die erfindungsgemäße Vorrichtung kann im Allgemeinen eine runde oder rundliche Form aufweisen. Ihr Durchmesser kann an den Anwendungsfall angepasst werden und bei Meerscheinchen beispielsweise einen Durchmesser von ca. 1 mm aufweisen, bei der Anwendung beim Menschen entsprechend ca. 1-15°mm größer. Die erfindungsgemäße Vorrichtung ist geeignet, das entsprechend damit behaftete vibrierfähige Gewebe, insbesondere ein Trommelfell, vor einem thermischen Schaden zu bewahren, welcher sich in einer Austrocknung gefolgt von einer Pigmentierung manifestiert. Insbesondere durch Austrocknung des vibrierfähigen Gewebes kann dieses in seiner mechanischen Eigenschaft nachteilig verändert, insbesondere geschwächt werden, wodurch Perforationen entstehen können. Durch die Verwendung der erfindungsgemäßen Vorrichtung zur optoakustischen Anregung des vibrierfähigen Gewebes kann eine dauerhaft schadenfreie mechanische Anregung des vibrierfähige Gewebes vorgenommen werden.

Nachfolgend werden Ausführungsformen der Erfindung anhand der beigefügten Zeichnungen beschrieben. Dabei zeigt
Figur 1 ein Ausführungsbeispiel der erfindungsgemäßen Vorrichtung,
Figur 2 ein Diagramm, in welchem ein Vergleich zwischen einer akustischen und einer optoakustischen Stimulation des Trommelfells veranschaulicht ist,
Fig. 3 ein Flussdiagramm, in dem eine Ausführungsform des Verfahrens zur mechanischen Anregung des Trommelfells oder einer weiteren vibrierfähigen Struktur des Schädels mittels elektromagnetischer Strahlung veranschaulicht ist,
Fig. 4A zeigt eine elektronenmikroskopische Aufnahme der für die Vorrichtung verwendbaren Trägerschicht, und
Fig. 4B zeigt eine lichtmikroskopische Aufnahme der für die Vorrichtung verwendbaren Trägerschicht.

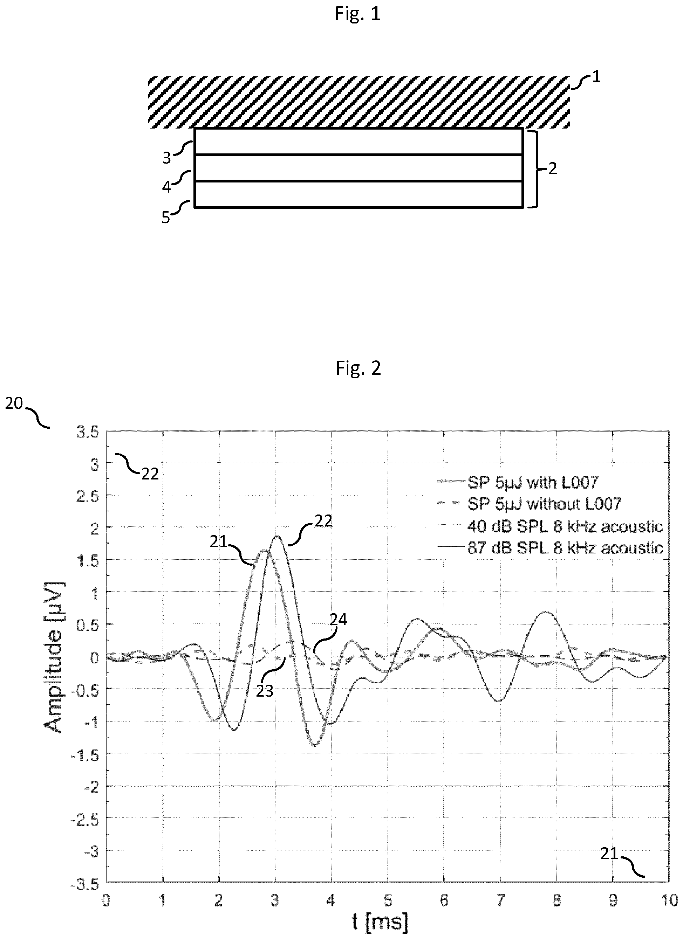

In **Fig. 1** ist eine beispielhafte erfindungsgemäße Vorrichtung 2 dargestellt. Im gezeigten Beispiel ist diese auf einem Trommelfell 1 angeordnet. Die Vorrichtung 2 weist eine Trägerschicht 3 auf. Die Vorrichtung 2 weist eine erste Substanz 4 auf, die eine erste Schicht ausbildet und bezüglich elektromagnetischer Strahlung mit einem vorbestimmen Wellenlängenspektrum eine reflektierende Eigenschaft aufweist. Ferner weist die Vorrichtung 2 eine zweite Substanz 5 auf, die eine zweite Schicht ausbildet, welche bezüglich elektromagnetischer Strahlung mit dem vorbestimmen Wellenlängenspektrum eine absorbierende Eigenschaft aufweist. Wie dargestellt, ist hierbei die erste Substanz 4 in einem Bereich zwischen der Trägerschicht 3 und der zweiten Substanz 5 angeordnet.

Eine optoakustische Anregung des Trommelfells 1 (oder, wie mehrfach erwähnt, einer anderen vibrierfähigen Struktur des Schädels) erfolgt mittels elektromagnetischer Strahlung, welche vom unteren Blattrand auf die Vorrichtung 2 auftrifft. Die elektromagnetische Strahlung trifft dabei zunächst auf die zweite Substanz 5 bzw. die entsprechende Materialschicht auf, die eingerichtet ist, die elektromagnetische Strahlung zu absorbieren. Derjenige Anteil der elektromagnetischen Strahlung, welcher nicht von der ersten Substanz 5 absorbiert worden ist und daher die entsprechende zweite Schicht passiert, wird von der ersten Substanz 4 bzw. der entsprechenden Materialschicht reflektiert und vor dem Durchdringen der Trägerschicht 3 und dem Eindringen ins tiefere Gewebe z. B Mittelohr gehindert. Die Vorrichtung 2 kann mittels eines Klebers oder eines anderen Adhäsivs an das Trommelfell 1 oder an eine weitere vibrierfähige Struktur des Schädels befestigt sein.

Die in Fig. 1 dargestellte Vorrichtung 2 kann beispielsweise hergestellt werden, indem zunächst die Trägerschicht 3 aus den Silikonelastomeren SSA MG 7-9800 und Sylgard 184 hergestellt wird. Dazu kann Sylgard 184, welches einen hohen elastischen Anteil besitzt und ein relativ rigides Verhalten zeigt, auf einen Trägerfilm aufgebracht und polymerisiert werden bei 95°C für 1h. Für die Einstellung der Schichtdicke kann eine automatische Rakel verwendet werden. Die Dicke des polymerisierten Films aus Sylgard 184 kann typischerweies 40 µm betragen. Anschließend kann MG 7-9800 auf den Film aus Sylgard 184 aufgebracht und ebenfalls polymerisiert werden, wobei eine typische Schichtdicke zwischen 40 µm und 80 µm liegen kann. Das MG 7-9800 Silikonelastomer zeichnet sich durch eine hohen viskose Komponente aus und kann zur Befestigung auf dem biologischen Gewebe, etwa dem Trommelfell, verwendet werden. In Versuchen konnte gezeigt werden, dass bei der Maus ein mehrfaches Aufbringen und erneutes Wiederablösen mit diesen Schichtkompositen möglich ist, ohne eine Perforation des Trommelfells herbeizuführen. Für die Ausbildung der Vorrichtung 2 kann das wie beschrieben ausgebildete zweischichtige Schichtkomposit von dem für deren Aufbau verwendeten Trägerfilm gelöst werden. Das Schichtkomposit kann dann als Trägerschicht 3 verwendet werden und es kann darauf die erste Substanz 4 und dann die zweite Substanz 5 angeordnet werden, beispielsweise in Form von entsprechenden Schichten.

Durch Verwenden der erfindungsgemäßen Vorrichtung zur optoakustischen Anregung eines biologischen Gewebes, insbesondere des Trommelfells, kann dieses nicht nur vor den nachteiligen Auswirkungen der dauerhaften Bestrahlung mit elektromagnetischer Strahlung bewahrt werden. Durch Verwenden der erfindungsgemäßen Vorrichtung kann primär im Vergleich zur direkten Bestrahlung des biologischen Gewebes eine Vergrößerung der Amplitude der resultierenden Gehöraktivierung erreicht werden. Dieser Aspekt ist in dem Diagramm 20 in **Fig. 2** veranschaulicht, in dem die natürliche akustische mit der optoakustischen Anregung des Trommelfells im Tiermodell (Meerschweinchen, Charles River) verglichen worden ist. Auf der x-Achse 21 ist die Zeit in Millisekunden aufgetragen und auf der y-Achse 22 ist die gemessene Amplitude in Mikrovolt aufgetragen. In dem Diagramm sind insgesamt vier Graphen 21-24 dargestellt. Der erste Graph 21 repräsentiert eine optoakustische Anregung des Trommelfells mittels eines anregenden optischen Pulses mit einer Energie von 5 µJ bei Verwendung der erfindungsgemäßen Vorrichtung auf dem Trommelfell. Der zweite Graph 22 repräsentiert eine natürliche Anregung des Trommelfells mit einem Schalldruck von 87 dB (ohne Verwendung der erfindungsgemäßen Vorrichtung). Der dritte Graph 21 repräsentiert eine optoakustische Anregung des Trommelfells mittels eines anregenden optischen Pulses mit einer Energie von 5 µJ ohne Verwendung der erfindungsgemäßen Vorrichtung, also bei einer direkten Bestrahlung des Trommelfells. Schließlich repräsentiert der vierte Graph 24 eine natürliche Anregung des Trommelfells mit einem Schalldruck von 40 dB (ohne Verwendung der erfindungsgemäßen Vorrichtung).

Durch Vergleich des ersten Graphen 21 mit dem dritten Graphen 23 sieht man, dass durch Verwenden der erfindungsgemäßen Vorrichtung eine erhebliche Steigerung der Anregungsamplitude im periphären Gehörsystem erreicht werden kann bei gleichbleibendem Anregungsimpuls. Der durch die Verwendung der erfindungsgemäßen Vorrichtung hervorgerufene Unterschied der Amplitude ist näherungsweise äquivalent zum Unterschied zwischen einer natürlichen Anregung des Gehörsystems mit einem Schalldruck von 40 dB (vierter Graph 24) und 87 dB (zweiter Graph 22). Die durch die Verwendung der erfindungsgemäßen Vorrichtung erzielbare Vergrößerung der oABR-Amplitude (opticallyevoked auditory brainstem response - optisch hervorgerufene Gehörreaktion des Hirnstamms) ist also signifikant. Insbesondere ermöglicht die Verwendung der Vorrichtung eine optoakustische Anregung der Hörbahn, welche mit einer natürlichen Anregung mit einem Schalldruckpegel oberhalb von 80 dB Schalldruck vergleichbar ist.

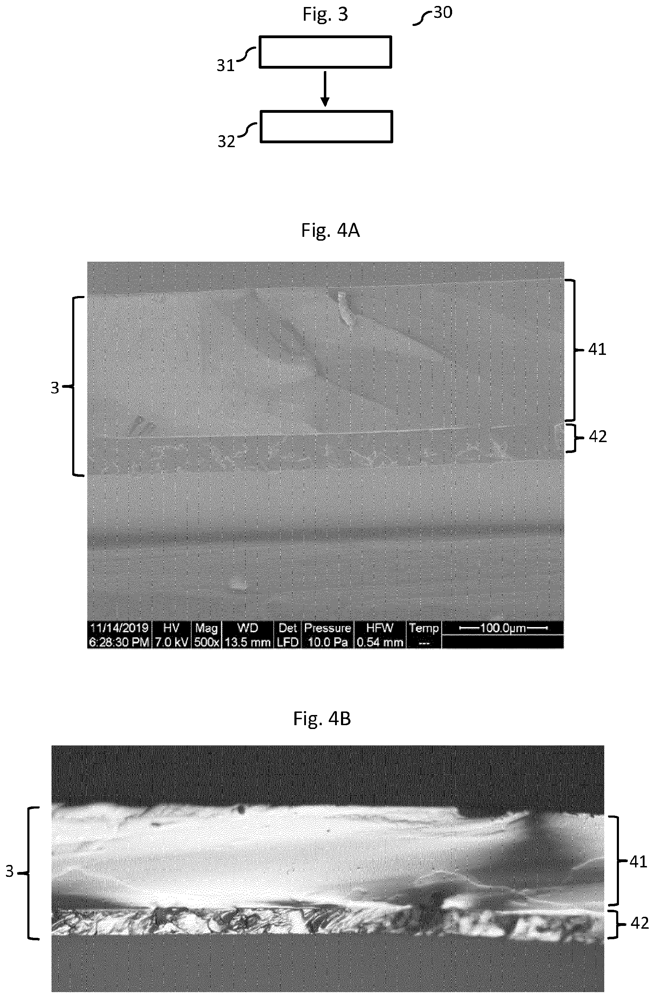

Fig. 3 zeigt ein Flussdiagramm 30, in dem eine Ausführungsform des Verfahrens zur mechanischen Anregung des Trommelfells oder einer weiteren vibrierfähigen Struktur des Schädels mittels elektromagnetischer Strahlung veranschaulicht ist. In einem ersten Schritt 31 weist das Verfahren Anbringen der erfindungsgemäßen Vorrichtung auf dass Trommelfell oder die weitere vibrierfähige Struktur des Schädels. In einem weiteren Schritt 32 weist das Verfahren Bestrahlen der Vorrichtung mit elektromagnetischer Strahlung mit dem vorbestimmten Wellenlängenspektrum.

In Fig. 4A ist eine elektronenmikroskopische Aufnahme eines Ausführungsbeispiels der Trägerschicht 3 dargestellt, welche für den Aufbau der erfindungsgemäßen Vorrichtung verwendet werden kann. Die Trägerschicht 3 weist einen zweischichtigen Aufbau auf, wobei eine erste Schicht 41 das Silikonelastomer SSA MG 7-9800 aufweist und die zweite Schicht 42 das Silikonelastomer Sylgard 184 aufweist. In dem gezeigten Beispiel hat die erste Schicht 41 eine Dicke von 158 µm und die zweite Schicht 42 hat eine Dicke von 42 µm. Die Trägerschicht 3 kann beispielsweise mittels Rotationsbeschichtung (spin coating) hergestellt werden, wobei die Dicken der einzelnen Schichten nach Bedarf eingestellt werden können.

In Fig. 4B ist eine lichtmikroskopische Aufnahme der in Fig. 4A gezeigten Trägerschicht 3 gezeigt.

Nach Fertigstellung der Trägerschicht 3 kann auf die Oberfläche der zweiten Schicht 42 die erste Substanz aufgebracht werden. Hierzu kann beispielsweise eine metallische Spiegelschicht aufgedampft werden. Nachfolgend kann die zweite Substanz aufgebracht werden, beispielsweise ein Farbstoff/Lack mittels eines Sprühverfahrens.

## Patentansprüche

1. Vibrierfähige Vorrichtung für das Gehörsystem, aufweisend:
eine Trägerschicht;
eine erste Substanz, welche bezüglich elektromagnetischer Strahlung mit einem vorbestimmen Wellenlängenspektrum eine reflektierende Eigenschaft aufweist;
eine zweite Substanz, welche bezüglich elektromagnetischer Strahlung mit dem vorbestimmen Wellenlängenspektrum eine absorbierende Eigenschaft aufweist;
wobei die erste Substanz in einem Bereich zwischen der Trägerschicht und der zweiten Substanz angeordnet ist.

2. Vorrichtung gemäß Anspruch 1,
wobei die Trägerschicht zwei aneinander anliegende Schichten aufweist.

3. Vorrichtung gemäß Anspruch 2, wobei die erste Schicht ein Silikonelastomer aufweist, bevorzugt SSA MG 7-9800, und die zweite Schicht ein Silikonelastomer aufweist, bevorzugt Sylgard 184.

4. Vorrichtung gemäß einem der Ansprüche 1 bis 3,
wobei die erste Substanz eine erste Funktionalschicht bildet, welche auf der Trägerschicht angeordnet ist.

5. Vorrichtung gemäß Anspruch 2 und 4,
wobei die erste Funktionalschicht auf der zweiten Schicht der Trägerschicht angeordnet ist.

6. Vorrichtung gemäß Anspruch 4 oder 5,
wobei die zweite Substanz eine zweite Funktionalschicht bildet, welche auf der ersten Funktionalschicht angeordnet ist.

7. Hörgerät, aufweisend:
die Vorrichtung gemäß einem der Ansprüche 1 bis 6;
einen Signalgeber, welcher eingerichtet ist, mittels mindestens einem Mikrophon Schall aufzunehmen und auf Basis des aufgenommenen Schalls elektromagnetische Strahlung mit dem vorbestimmen Wellenlängenspektrum zu emittieren.

8. Vorrichtung gemäß einem der Ansprüche 1 bis 6 zur Verwendung in einem Verfahren zur mechanischen Anregung des Trommelfells mittels elektromagnetischer Strahlung.

## Claims

1. Vibration-capable apparatus for the auditory system, comprising:
a carrier layer;
a first substance which has a reflective property with respect to electromagnetic radiation with a predetermined wavelength spectrum;
a second substance which has an absorbing property with respect to electromagnetic radiation with the predetermined wavelength spectrum;
where the first substance is arranged in an area between the carrier layer and the second substance.

2. The apparatus according to claim 1,
whereby the carrier layer has two layers adjacent to each other.

3. The apparatus according to claim 2, wherein the first layer has a silicone elastomer, prefers SSA MG 7-9800, and the second layer has a silicone elastomer, preferably Sylgard 184.

4. The apparatus according to any one of claims 1 to 3,
where the first substance forms a first functional layer, which is arranged on the carrier layer.

5. The apparatus according to claims 2 and 4,
where the first functional layer is arranged on top of the second layer of the carrier layer.

6. The apparatus according to claim 4 or 5,
where the second substance forms a second functional layer, which is arranged on top of the first functional layer.

7. Hearing aid, having:
the apparatus according to any one of claims 1 to 6;
a signal transmitter that is set up to pick up sound by means of at least one microphone and to emit electromagnetic radiation with the predetermined wavelength spectrum on the basis of the recorded sound.

8. An apparatus according to any one of claims 1 to 6 for use in a method for mechanical stimulation of the eardrum by means of electromagnetic radiation.

## Revendications

1. Vibration-capable dispositif pour le système auditif, comprenant:
une couche de support;
une première substance qui a une propriété réfléchissante par rapport au rayonnement électromagnétique avec un spectre de longueurs d'onde prédéterminé;
une deuxième substance qui a une propriété absorbante par rapport au rayonnement électromagnétique avec le spectre de longueurs d'onde prédéterminé;
où la première substance est disposée dans une zone située entre la couche porteuse et la seconde substance.

2. Dispositif selon la revendication 1,
la couche porteuse a deux couches adjacentes l'une à l'autre.

3. Dispositif selon la revendication 2, dans lequel la première couche est dotée d'un élastomère de silicone, préfère le SSA MG 7-9800, et la seconde couche a un élastomère de silicone, de préférence Sylgard 184.

4. Dispositif selon l'une quelconque des revendications 1 à 3,
où la première substance forme une première couche fonctionnelle, qui est disposée sur la couche porteuse.

5. Dispositif selon les revendications 2 et 4,
où la première couche fonctionnelle est disposée au-dessus de la deuxième couche de la couche porteuse.

6. Dispositif selon la revendication 4 ou 5,
où la seconde substance forme une seconde couche fonctionnelle, qui est disposée au-dessus de la première couche fonctionnelle.

7. Prothèse auditive ayant :
le dispositif selon l'une quelconque des revendications 1 à 6;
Émetteur de signal configuré pour capter le son au moyen d'au moins un microphone et pour émettre un rayonnement électromagnétique avec le spectre de longueurs d'onde prédéterminé sur la base du son enregistré.

8. Dispositif selon l'une quelconque des revendications 1 à 6, destiné à être utilisé dans une méthode de stimulation mécanique du tympan au moyen d'un rayonnement électromagnétique.
